# EUROPEAN PATENT APPLICATION

(11) **EP 4 763 242 A1**
(43) Date of publication of application: **24.06.2026**
(21) Application number: 25225584.9
(22) Date of filing: 19.12.2025
(51) Int. Cl.: A61M 1/16, A61M 1/36, A61M 39/12, A61M 60/109, A61M 60/232, A61M 60/38, A61M 60/859

(54) **ELASTIC SLEEVE FOR COUPLING FLEXIBLE TUBING TO A MEDICAL DEVICE**

(30) Priority: 20.12.2024 US 202418990897
(71) Applicant: Lifemotion Medical Technology Co., Ltd., Shenzhen, Guangdong 518102 (CN)
(72) Inventor: ZANIBONI, Andrea, 41037 Mirandola (IT)
(74) Representative: Pfenning, Meinig & Partner mbB

(57) **Abstract**

A coupling system includes a connector having an external surface with an outer diameter. The connector has a coupling portion with an external surface and an outer diameter. The coupling portion includes a coupling length extending proximally from a connector distal end. A flexible tubing includes an inner diameter configured to fit over the outer diameter. An elastic element has a lumen configured to receive both the connector and the flexible tubing. The elastic element is configured to squeeze the flexible tubing onto the connector along the entire coupling length.

## Description

### TECHNICAL FIELD

The present disclosure relates to the field of medical devices. More specifically, the present disclosure relates to systems and methods for coupling flexible tubing to medical devices.

### BACKGROUND

Extracorporeal circulation during cardiac surgery and extracorporeal membrane oxygenation (ECMO) applications is managed by means of closed loop circuits. In general, portions of the circuits are made of flexible and transparent polyvinyl chloride (PVC) or polyurethane (PU) tubes connecting various components together. For example, venous/arterial cannula, pumps, oxygenators, and other devices like sensors for measuring pressures or other blood parameters, are joined by flexible tubes or tubing forming closed loop circuits. These components are usually made of rigid and transparent polycarbonate. Barbed connectors are often present at the ends of their inlet/outlet conduits and have dimensions chosen according to the maximum circulating blood flow rates. For instance, in extracorporeal circuits for adult use, the connectors typically have inner diameters of three-eight inch while in pediatric devices they are typically one-quarter inch.

Polycarbonate connector external diameters and their barb shape/positions depend on the manufacturer, while PVC tubes are generally characterized by a wall thickness of about 2.3 - 2.5 mm. Coupling between PVC tubes and polycarbonate connectors is made with interference to ensure sealing during use with blood (for instance, in the adult tubing sets the maximum interference is kept in the range of 3 - 4 mm).

To make the connections more consistent and safer, manufacturers commonly use plastic tie-wraps of the same type utilized for electric wiring. The tie-wrap is placed around the outside of the flexible tube and its latch is tightened with a certain force.

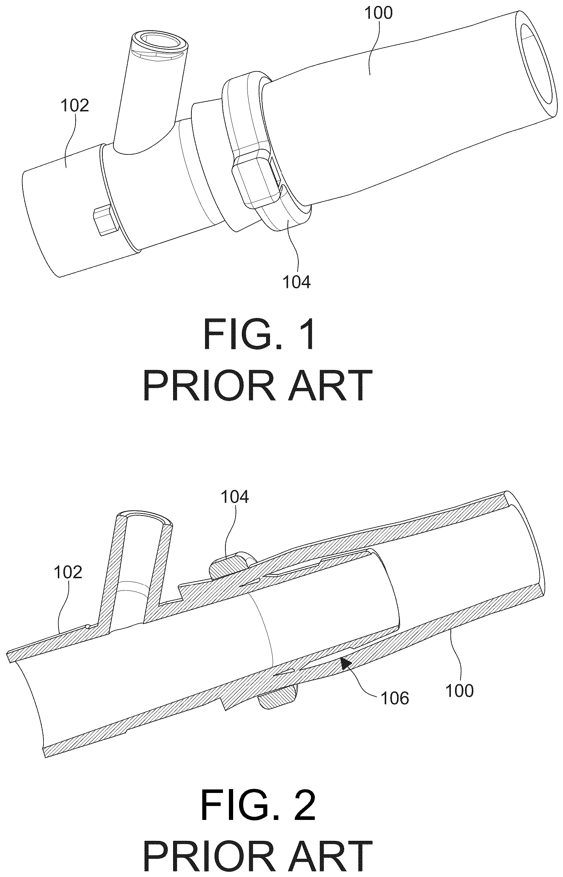

FIGS. 1 and 2 show an example of a prior art connection between a flexible PVC tube 100 and the extremity of a polycarbonate (PC) connector 102 utilizing a tie-wrap 104 for securing the tube 100 to the connector 102.

Flexible PVC and rigid PC, the two constituent materials of the elements to be connected, are not chemically compatible and cannot be bonded together. In some instances, an appropriate blend of volatile PVC and PC solvents may be used in addition to the tie-wrap 104 to strengthen the coupling between the flexible tube 100 and the connector 102. In this way, the resulting connection is very tight and resistant, but its geometry still presents a problem. As illustrated in FIG. 2, one or more small gaps 106 between an inner diameter of the tube 100 and an outer diameter of the connector 102 provide a location where blood may infiltrate and stagnate, thus creating local blood clots which then may release microemboli into blood circulation, which is extremely dangerous for the patient. The one or more gaps 106 can surround the connector 102 due to a non-perfect fit of the tube 100 inner diameter to the connector 102. The one or more gaps 106 can also increase in size as a result of blood temperature and procedure duration, both responsible for causing a certain degradation of tube 100 mechanical properties. This problem is more likely to happen during prolonged ECC (Extra Corporeal Circulation) procedures.

The use of a tie-wrap 104 does not bring particular benefits to the sealing characteristics of the connection between the connector 102 and the flexible tubing 100. The tie-wrap 104 merely exerts a concentrated pressure in a very limited portion (3 - 3.5 mm wide) of a total coupling length (20 - 25 mm) of the connector 102. The tie-wrap 104 may also be subjected to relaxation over time and be the cause of blood leaks as the tie-wrap 104 weakens, particularly in the area below the tie-wrap 104.

### SUMMARY

In order to solve or alleviate the problems associated with coupling using a tie-wrap, the solution discussed herein implies to abandon the tie-wrap in favor of an elastic element. The elastic element provides homogenous radial and longitudinal pressure, throughout an entire coupling length, on a flexible tube wall against the connector to avoid the formation of gaps between the two components. No gaps result in an absence of blood stagnation areas and in no blood clot formation. Additionally, the elastic element maintains the pressure around the flexible tube coupling portion constant overtime without any relaxation. Such a solution is found in the following examples:
Example 1 includes a coupling system. The system includes a connector having an external surface with an outer diameter. The connector has a coupling portion with an external surface and an outer diameter. The coupling portion includes a coupling length extending proximally from a connector distal end. A flexible tubing includes an inner diameter configured to fit over the outer diameter. An elastic element has a lumen configured to receive both the connector and the flexible tubing. The elastic element is configured to squeeze the flexible tubing onto the connector along the entire coupling length.
Example 2 is the system of Example 1, wherein the connector includes at least one barb having an outer diameter larger than the external surface.
Example 3 is the system of Example 1, wherein the connector is rigid.
Example 4 is the system of Example 1, wherein the elastic element has a first end and a second end, the first end and the second end separated by a length at least equal to the coupling length.
Example 5 is the system of Example 1, wherein the elastic element has a first outer diameter along a first portion, and a second diameter along a second portion, the first diameter being larger than the second diameter.
Example 6 is the system of Example 1, wherein the elastic element includes at least one groove extending into an inner surface.
Example 7 is the system of Example 1, wherein the elastic element comprises an elastomeric material.
Example 8 is the system of Example 1, wherein the elastic element comprises silicone or rubber.
Example 9 is the system of Example 1, wherein the elastic element is a sleeve and applies constant pressure over the entire coupling length.
Example 10 is the system of Example 1, wherein the elastic element has a length in the range of 10 mm to 30 mm, a wall thickness in the range of 1.5 mm to 3.5 mm, and a hardness in the range of 30 to 60 Shore A.
Example 11 is a closed loop circuit for use in a medical procedure. The circuit includes an oxygenator having a rigid connector. The connector has a coupling portion with an external surface and an outer diameter. The coupling portion includes a coupling length extending proximally from a connector distal end. A flexible tubing includes an inner diameter configured to fit over the outer diameter. An elastic element includes a lumen configured to receive both the connector and the flexible tubing. The elastic element is configured to squeeze the flexible tubing onto the connector along the entire coupling length.
Example 12 is the circuit of Example 11, wherein the connector includes at least one barb having an outer diameter larger than the external surface.
Example 13 is the circuit of Example 11, wherein the elastic element has a first end and a second end, the first end and the second end separated by a length at least equal to the coupling length.
Example 14 is the circuit of Example 11, wherein the elastic element has a first outer diameter along a first portion, and a second diameter along a second portion, the first diameter being larger than the second diameter.
Example 15 is the circuit of Example 11, wherein the elastic element includes at least one groove extending into an inner surface.
Example 16 is the circuit of Example 11, wherein the elastic element comprises an elastomeric material.
Example 17 is the circuit of Example 11, wherein the elastic element comprises silicone or rubber.
Example 18 is the circuit Example 11, wherein the elastic element is a sleeve and applies constant pressure over the entire coupling length.
Example 19 is the circuit of Example 11, wherein the elastic element has a length in the range of 10 mm to 30 mm, a wall thickness in the range of 1.5 mm to 3.5 mm, and a hardness in the range of 30 to 60 Shore A.
Example 20 is a method for coupling a flexible tube to a connector. The method includes expanding an elastic element having a lumen. The method includes placing the elastic element over a portion of the flexible tube, so the portion of the flexible tube is located in the lumen. The method includes introducing a connector into a lumen of the flexible tubing, wherein a coupling portion of the connector is surrounded by the portion of the flexible tube and the elastic element. The elastic element squeezes the flexile tubing onto the connector along the coupling portion to prevent gaps between the flexible tube and the connector.

### BRIEF DESCRIPTION OF THE DRAWINGS

The drawings described here are used to provide a further understanding of the present application and constitute a part of the present application. The illustrative embodiments of the present application and their descriptions are used to explain the present application and do not constitute an improper limitation of the present application. Some specific embodiments of the present application will be described in detail below by way of illustration and not limitation with reference to the accompanying drawings. In the drawings, like reference numerals designate the same or similar components or portions. It will be understood by those skilled in the art that these drawings are not necessarily drawn to scale. In the drawings:
FIG. 1 is a perspective view of a prior art coupling between a flexible tubing and a rigid connector using a tie-wrap;
FIG. 2 is a three-dimensional cross-sectional view of the prior art coupling in FIG. 1 between a flexible tubing and a rigid connector using a tie-wrap;
FIG. 3 is an exploded perspective view of components for coupling between a flexible tubing and a rigid connector using an elastic element, according to embodiments of the present disclosure;
FIG. 4 is a cross-sectional view of an elastic element having more than one inner and/or outer diameter, according to embodiments of the present disclosure;
FIG. 5 is a cross-sectional view of an elastic element having at least one groove extending into inner surface thereof, according to embodiments of the present disclosure;
FIG. 6 is a perspective view of an elastic element loaded onto a flexible tubing, according to embodiments of the present disclosure;
FIG. 7 is a perspective view of a coupling between a flexible tubing and a rigid connector using an elastic element, according to embodiments of the present disclosure; and
FIG. 8 is a perspective view of an oxygenator utilizing an elastic element for coupling between a flexible tubing and a rigid connector, according to embodiments of the present disclosure.

While the disclosure is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the disclosure as defined by the appended claims.

### DETAILED DESCRIPTION

For purposes of promoting an understanding of the principles of the present disclosure, reference is now made to the examples illustrated in the drawings, which are described below. The illustrated examples disclosed herein are not intended to be exhaustive or to limit the disclosure to the precise form disclosed in the following detailed description. Rather, these exemplary embodiments were chosen and described so that others skilled in the art may use their teachings. It is not beyond the scope of this disclosure to have a number (e.g., all) the features in a given example used across all examples. Thus, no one figure should be interpreted as having any dependency or requirement related to any single component or combination of components illustrated therein. Additionally, various components depicted in a given figure may be, in examples, integrated with various ones of the other components depicted therein (and/or components not illustrated), all of which are considered to be within the ambit of the present disclosure.

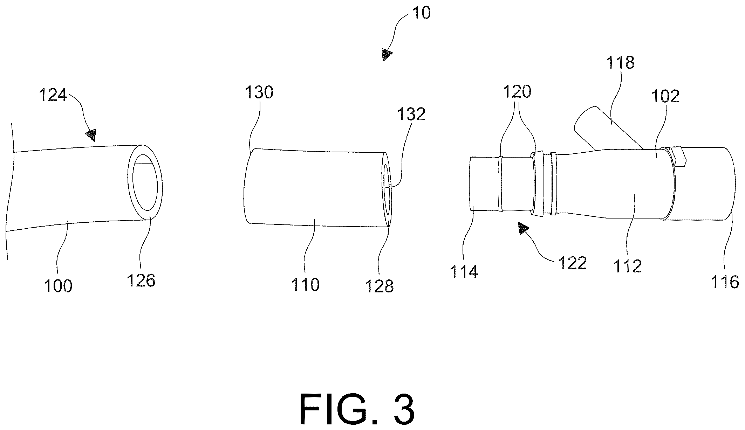

FIG. 3 illustrates the various components of a system 10 for coupling a flexible tubing 100 (also referred to as a flexible tube) to a rigid connector 102 using an elastic element 110. In some embodiments, the rigid connector 102 is separate from but configured to join a medical device such as a cannula, pump, oxygenator, or sensor. In other embodiments, the rigid connector 102 is integral with a medical device.

The rigid connector 102 includes a body 112 having a distal end 114 and an opposite proximal end 116. The rigid connector 102 in FIG. 3 is not integral with a medical device, as such, the proximal end 116 is configured to join with a medical device. The body 112 includes a port 118 that is configured for connecting with a sensor, flexible tubing, or other medical device necessary for performing extracorporeal circulation. In some embodiments, the rigid connector 102 does not include a port 118. As illustrated in FIG. 3, the port 118 is at an angle with respect to the body 112. In some embodiments, the port 118 is perpendicular to the body 112.

The connector 102 includes a coupling portion 122 configured to receive a proximal portion 124 of the flexible tubing 100. The coupling portion 122 has an external surface having an outer diameter that is larger than an inner diameter of the flexible tubing 100. The flexible tubing 100 is configured to expand or stretch in the proximal portion 124 so the inner diameter fits over the outer diameter of the coupling portion 122.

The coupling portion 122 includes at least one barb 120 configured to help secure the flexible tubing 100 to the connector 102. The at least one barb 120 includes an outer diameter that is larger than an outer diameter of the external surface of the coupling portion 122 adjacent the at least one barb 120.

The coupling portion 122 defines a coupling length. The coupling length extends proximally from the distal end 114 to a location along the coupling portion 122 that corresponds to a flexible tubing 100 proximal end 126. In other words, the coupling length correlates to a length of the coupling portion 122 that is received within the flexible tubing 100. The coupling length includes the at least one barb 120.

The elastic element 110 has a proximal end 128, a distal end 130, and a lumen 132 extending between the proximal end 128 and the distal end 130. The lumen 132 is configured to fit over and receive both the connector 102 and the flexible tubing 100. The lumen 132 includes an inner diameter that is smaller than an outer diameter of the proximal portion 124 of the flexible tubing 100. The lumen 132 has a shape that corresponds to the external surface of the coupling portion 122. For example, if the coupling portion 122 is cylindrical, then the lumen 132 has a circular cross-section. Similarly, in some embodiments, the coupling portion 122 and the lumen 132 may have corresponding non-circular shapes.

The elastic element 110 has a length extending between the proximal end 128 and the distal end 130 that is at least as long as the coupling length of the coupling portion 122. In some embodiments, the length between the proximal end 128 and the distal end 130 is equal to the coupling length of the coupling portion 122. The elastic element is configured to squeeze the flexible tubing 100 onto the connector 102 along the entire coupling length in order to prevent gaps forming between the connector 102 and the inner surface of the flexible tubing 100.

The elastic element 110 is configured to expand to receive the flexible tubing 100 and the connector 102, then to apply a constant pressure over the entire coupling length of the coupling portion 122 when released. The elastic element 110 comprises an elastomeric material capable of applying a constant circumferential pressure to the flexible tubing 100 and the connector 102 over time, without degradation. In some embodiments, the elastic element 110 is made of silicone, which provides desirable elastic properties, but does not degrade and is not influenced by effects of either temperature or duration time of a procedure. In other embodiments, the elastic element 110 is made of rubber.

In some embodiments, the elastic element 110 takes the form of a sleeve having a constant outer diameter and a constant inner diameter along the lumen 132. In some embodiments, the elastic element 110 has a length between the proximal end 128 and the distal end 130 in the range of 10 mm to 30 mm. In some embodiments, the elastic element 110 has a wall thickness in the range of 1.5 mm to 3.5 mm. In some embodiments, the elastic element 110 is formed of a material having a hardness in the range of 30 to 60 Shore A.

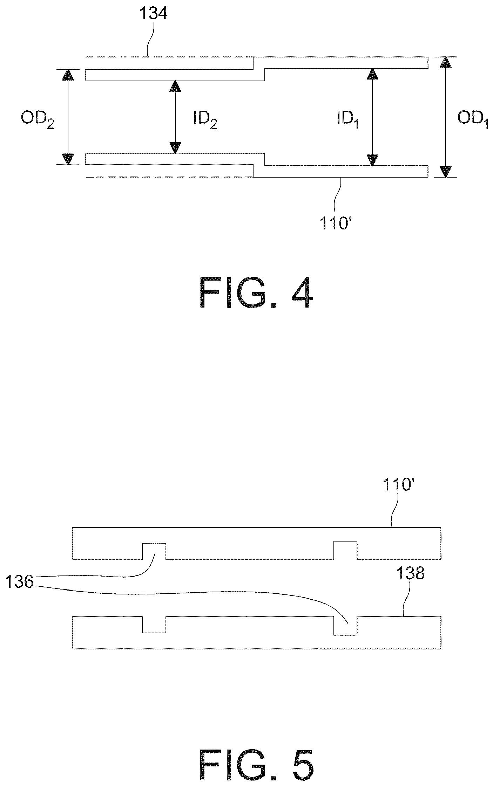

In some embodiments, the elastic element may have more than one inner and outer diameter along a length thereof. For example, FIG. 4 is a cross-section of an elastic element 110' having a first inner diameter ID₁ along a first portion and a second inner diameter ID₂ along a second portion. The first inner diameter ID₁ is larger than the second inner diameter ID₂. A first outer diameter OD₁ is also larger than a second outer diameter OD₂. In some embodiments, as noted by phantom line 134, the outer diameter of the elastic element 110' can remain constant while the inner diameter varies.

FIG. 5 illustrates a cross-section of an elastic element 110" having at least one groove 136 located within the inner surface 138 of the lumen 132. In some embodiments, the location of the at least one groove 136 corresponds to the at least one barb 120 on the coupling portion 122. For example, the spacing between a plurality of grooves 136 within the elastic element 110" is equal to the spacing between a plurality of barbs 120 on the coupling portion 122, such that the plurality of grooves 136 overlie the plurality of barbs 120 when the elastic element 110" is positioned over the coupling portion 122.

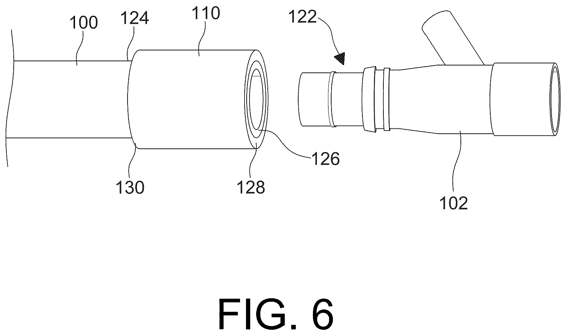

FIG. 6 is a perspective view of an elastic element 110 loaded onto a flexible tubing 100 prior to connection with a connector 102. In order for the elastic element 110 to be placed on the flexible tubing 100, the elastic element is expanded using a tube expander and placed over the proximal portion 124 of the flexible tubing 100. As illustrated, the proximal end 126 of the flexible tubing 100 and the proximal end 128 of the elastic element 110 are in alignment with one another.

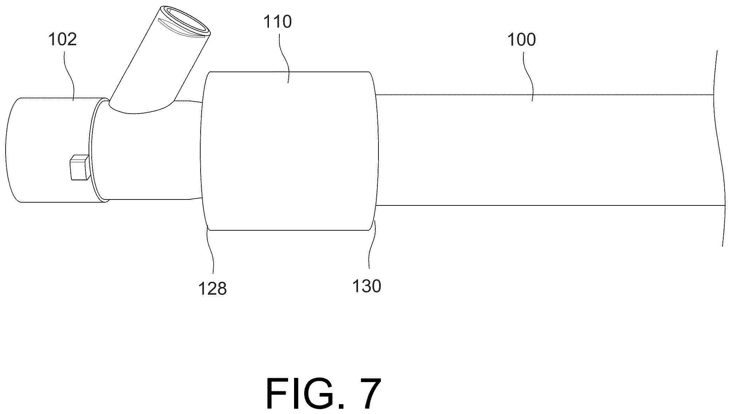

At this point, the coupling portion 122 of the connector 102 is manually introduced into the flexible tubing 100. This action can be facilitated by the presence of the elastic element 110 which offers more finger grip for a user. FIG. 7 shows the final result, where the elastic element 110 covers and forces the flexible tubing 100 against the connector along the entire coupling length of the coupling portion 122. By this arrangement, the flexible tube 100 is forced into intimate contact with the coupling portion 122 of connector 102, thereby eliminating internal gaps or recesses between the two (even when the tube is bent).

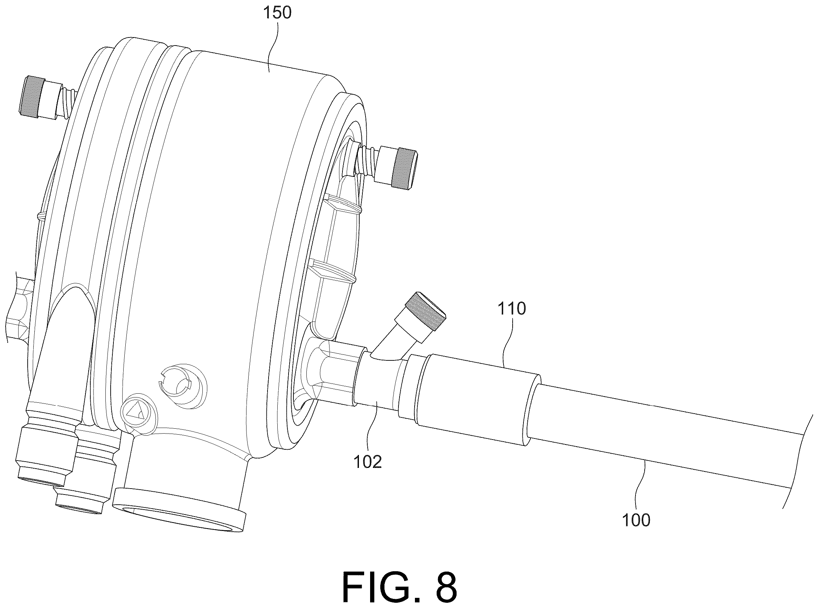

FIG. 8 is a perspective view of an oxygenator 150 utilizing an elastic element 110 for coupling between a flexible tubing 100 and a rigid connector 102. In FIG. 8, the oxygenator 150 is a stacked layer oxygenator for use in ECMO applications. A closed loop circuit is formed between the oxygenator 150 and a patient (not shown) utilizing the flexible tubing 100 to form a portion of the loop. The elastic element 110 surrounds both the flexible tubing 100 and a coupling portion 122 of the connector 102 to ensure no gaps are formed between the flexible tubing 100 and the connector 102 as discussed above.

Finally, it should be noted that the above embodiments are only used to illustrate the technical solution of the present application, but not to limit it; although the present application has been described in detail with reference to the foregoing embodiments, those of ordinary skill in the art should understand that the technical solutions described in the foregoing embodiments can still be modified, or some or all of the technical features can be equivalently replaced, and these modifications or substitutions do not deviate from the essence of the corresponding technical solutions from the technical solutions of the embodiments of the present application.

It is well understood that methods that include one or more steps, the order listed is not a limitation of the claim unless there are explicit or implicit statements to the contrary in the specification or claim itself. It is also well settled that the illustrated methods are just some examples of many examples disclosed, and certain steps may be added or omitted without departing from the scope of this disclosure. Such steps may include incorporating devices, systems, or methods or components thereof as well as what is well understood, routine, and conventional in the art.

The connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements. The scope is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B or C may be present in a single embodiment; for example, A and B, A and C, B and C, or A and B and C. The terms "couples," "coupled," "connected," "attached," and the like along with variations thereof are used to include both arrangements wherein two or more components are in direct physical contact and arrangements wherein the two or more components are not in direct contact with each other (e.g., the components are "coupled" via at least a third component), but still cooperate or interact with each other.

In the detailed description herein, references to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art with the benefit of the present disclosure to affect such feature, structure, or characteristic in connection with other embodiments whether or not explicitly described. After reading the description, it will be apparent to one skilled in the relevant art(s) how to implement the disclosure in alternative embodiments.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

## Claims

1. A coupling system, the system comprising:
a connector having a coupling portion with an external surface having an outer diameter, the coupling portion having a coupling length extending proximally from a connector distal end;
a flexible tubing having an inner diameter configured to fit over the outer diameter; and
an elastic element having a lumen configured to receive both the connector and the flexible tubing, the elastic element configured to squeeze the flexible tubing onto the connector along the entire coupling length.

2. The system of claim 1, wherein
the connector includes at least one barb having an outer diameter larger than the external surface, and/or
the connector is rigid.

3. The system of claim 1 or 2, wherein
the elastic element has a first end and a second end, the first end and the second end separated by a length at least equal to the coupling length, and/or
the elastic element has a first outer diameter along a first portion, and a second diameter along a second portion, the first diameter being larger than the second diameter.

4. The system of one of claims 1 to 3, wherein
the elastic element includes at least one groove extending into an inner surface.

5. The system of one of claims 1 to 4, wherein
the elastic element comprises an elastomeric material, and/or
the elastic element comprises silicone or rubber.

6. The system of one of claims 1 to 5, wherein
the elastic element is a sleeve and applies constant pressure over the entire coupling length.

7. The system of one of claims 1 to 6, wherein
the elastic element has a length in the range of 10 mm to 30 mm, a wall thickness in the range of 1.5 mm to 3.5 mm, and a hardness in the range of 30 to 60 Shore A.

8. A closed loop circuit for use in a medical procedure, the circuit comprising:
an oxygenator having a rigid connector, a connector having a coupling portion with an external surface having an outer diameter, the coupling portion having a coupling length extending proximally from a connector distal end;
a flexible tubing having an inner diameter configured to fit over the outer diameter; and
an elastic element having a lumen configured to receive both the connector and the flexible tubing, the elastic element configured to squeeze the flexible tubing onto the connector along the entire coupling length.

9. The circuit of claim 8, wherein
the connector includes at least one barb having an outer diameter larger than the external surface, and/or
the elastic element has a first end and a second end, the first end and the second end separated by a length at least equal to the coupling length.

10. The circuit of claim 8 or 9, wherein
the elastic element has a first outer diameter along a first portion, and a second diameter along a second portion, the first diameter being larger than the second diameter, and/or
the elastic element includes at least one groove extending into an inner surface.

11. The circuit of one of claims 8 to 10, wherein
the elastic element comprises an elastomeric material.

12. The circuit of one of claims 8 to 11, wherein
the elastic element comprises silicone or rubber.

13. The circuit of one of claims 8 to 12, wherein
the elastic element is a sleeve and applies constant pressure over the entire coupling length.

14. The circuit of one of claims 8 to 13, wherein
the elastic element has a length in the range of 10 mm to 30 mm, a wall thickness in the range of 1.5 mm to 3.5 mm, and a hardness in the range of 30 to 60 Shore A.

15. A method for coupling a flexible tube to a connector, the method comprising:
expanding an elastic element having a lumen;
placing the elastic element over a portion of the flexible tube so the portion of the flexible tube is located in the lumen; and
introducing a connector into a lumen of the flexible tubing, wherein a coupling portion of the connector is surrounded by the portion of the flexible tube and the elastic element; wherein the elastic element squeezes the flexile tubing onto the connector along the coupling portion to prevent gaps between the flexible tube and the connector.
